(19) Europäisches Patentamt
European Patent Office
Office européen des brevets

(11) Veröffentlichungsnummer: 0 296 441
A2

(12) # EUROPÄISCHE PATENTANMELDUNG

(21) Anmeldenummer: 88109356.1

(22) Anmeldetag: 13.06.88

(51) Int. Cl.4: A01N 25/12 , C02F 1/50 , A61L 15/03 , A61L 9/00 , A01N 33/12

(30) Priorität: 22.06.87 DE 3720555

(43) Veröffentlichungstag der Anmeldung: 28.12.88 Patentblatt 88/52

(84) Benannte Vertragsstaaten: AT BE CH DE ES FR GB IT LI NL SE

(71) Anmelder: Henkel Kommanditgesellschaft auf Aktien
Postfach 1100 Henkelstrasse 67
D-4000 Düsseldorf 1(DE)

(72) Erfinder: Lehmann, Rudolf, Dr.
Schnugsheide 2
D-5653 Leichlingen 1(DE)
Erfinder: Engelskirchen, Konrad, Dr.
Gonellastrasse 24
D-4005 Meerbusch 3(DE)
Erfinder: Grebe, Hans-Joachim
Moltkestrasse 61
D-4000 Düsseldorf 30(DE)
Erfinder: Meffert, Alfred, Dr.
Marie-Curie-Strasse 10
D-4019 Monheim(DE)
Erfinder: Syldatk, Andreas, Dr.
Am Nettchesfeld 25
D-4000 Düsseldorf 13(DE)
Erfinder: Fues, Johann-Friedrich, Dr.
Otto-Hahn-Strasse 157
D-4000 Düsseldorf(DE)

(54) **Verwendung von unlöslichen, polyfunktionellen quartären Ammoniumverbindungen zur adsorptiven Bindung von Mikroorganismen.**

(57) Die Erfindung betrifft die Verwendung von unlöslichen und/oder auf einem unlöslichen Träger immobilisierten polyfunktionellen, quartären Ammoniumverbindungen zur adsorptiven Bindung von Mikroorganismen, insbesondere Hefen und/oder Bakterien. Bevorzugte Anwendungsbereiche sind: die Entkeimung von Flüssigphasen, insbesondere von wäßrigen Flüssigkeiten, von Gasphasen, insbesondere Raumluft, und von harten Oberflächen beziehungsweise die Entkeimung verletzter und unverletzter Bereiche des menschlichen oder tierischen Körpers, insbesondere zur Wundversorgung.

EP 0 296 441 A2

## "Verwendung von unlöslichen, polyfunktionellen quartären Ammoniumverbindungen zur adsorptiven Bindung von Mikroorganismen"

Gegenstand der älteren Anmeldung P 36 44 579.7 (D 7816) sind neue Flockungs- und Filtrierhilfsmittel zur erleichterten Abtrennung von Trübstoffen und partikulären Feststoffen aus Flüssigphasen auf Basis polykationischer Polymerverbindungen, wobei das Kennzeichen dieser Hilfsmittel darin liegt, daß sie in den zu klärenden Flüssigphasen wenigstens weitgehend unlösliche Feststoffe sind, die wenigstens an ihrer Oberfläche eine polykationische Ausrüstung aufweisen, die bevorzugt flockungsaktive quartäre Ammoniumgruppen sind. Diese Flockungs- und Filtrierhilfsmittel sollen dabei als Feststoffpartikel solcher Größe vorliegen, daß eine Mehrzahl der abzutrennenden Trübstoffteilchen beziehungsweise partikulären Verunreinigungen auf der Oberfläche eines polykationischen Partikels gebunden werden kann.

Beschrieben wird in der genannten älteren Anmeldung insbesondere die Verwendung dieser Flockungs- und Filtrierhilfsmittel zur Klärung von Flüssigphasen insbesondere wäßrigen Phasen, die gelöste und ungelöste anorganische und/oder organische Komponenten enthalten, wobei optisch praktisch klare Flüssigphasen erhalten werden können.

Die vorliegende Erfindung geht von der Erkenntnis aus, daß sich die Flockungs- und Filtrierhilfsmittel der zuvor genannten älteren Anmeldung insbesondere auch für den Einsatz auf einem Sondergebiet eignen, das im nachfolgenden geschildert wird.

Gegenstand der vorliegenden Erfindung ist dementsprechend die Verwendung von unter Einsatzbedingungen unlöslichen und/oder auf einem unlöslichen Träger immobilisierten polyfunktionellen quartären Ammoniumverbindungen (der Einfachheit halber im nachfolgenden mit PQAV bezeichnet) zur adsorptiven Bindung von Mikroorganismen d.h. zur Bindung von Keimen unterschiedlichsten Ursprungs. Besonders eignet sich die Lehre der Erfindung für die adsorptive Bindung von Hefen und/oder Bakterien beliebigen Ursprungs. Das adsorptive Bindungsvermögen der erfindungsgemäß eingesetzten PQAV besteht dabei sowohl gegenüber dem lebenden wie gegenüber dem abgestorbenen beziehungsweise abgetöten Keim und/oder seinen Restprodukten.

Es leuchtet ein, daß insbesondere die Bindungsfähigkeit der erfindungsgemäß eingesetzten unlöslichen beziehungsweise immobilisierten PQAV gegenüber lebenden Mikroorganismen breite Anwendungsgebiete für das erfindungsgemäße Handeln eröffnet. So können die unlöslichen und/oder immobilisierten PQAV zur Entkeimung von beziehungsweise Keimreduzierung in Flüssigphasen eignesetzt werden, wobei hier wiederum wäßrigen Flüssigkeiten besondere Bedeutung zukommen kann. Die Bindung der Mikroorganismen an die unlöslichen PQAV hat im Sinne der Erfindung aber auch Bedeutung beispielsweise für die Entkeimung von Gasphasen insbesondere für die entsprechende Behandlung von Luft, deren Keimgehalt dadurch beträchtlich gesenkt werden kann, daß der verkeimte Luftstrom durch geeignete Filterbetten geleitet wird, die mit den erfindungsgemäß vorgesehenen unlöslichen beziehungsweise immobilisierten PQAV ausgerüstet sind.

Ein weiteres Einsatzgebiet für die adsorptive Bindung von Mikroorganismen insbesondere Hefen und/oder Bakterien liegt in der Behandlung harter Oberflächen neben und/oder anstelle üblicher desinfizierender Behandlungsschritte für entsprechende verkeimte Flächenbereiche.

Ein besonders wichtiges Einsatzgebiet für die erfindungsgemäße Lehre findet sich in der Entkeimung verletzter oder unverletzter Bereiche des menschlichen oder tierischen Körpers und hierbei insbesondere in der Wundversorgung am menschlichen und tierischen Körper. Hier kann durch unlösliche beziehungsweise auf einem unlöslichen Träger immobilisierte PQAV die verbesserte Entsorgung bereits verkeimter Wundbereiche ebenso sichergestellt werden wie ein verbesserter Schutz gegen der Zutritt von Mikroorganismen, insbesondere der genannten Art, in den Wundbereich hinein.

Diese Vielgestaltigkeit der Anwendungsmöglichkeiten der erfindungsgemäß definierten PQAV für das erfindungsgemäße Ziel der adsorptiven Bindung von Mikroorganismen beziehungsweise Keimen macht wiederum verständlich, daß die im jeweiligen Einsatzfall zu verwendenden PQAV-Feststoffkörper in unterschiedlichster Formgestaltung vorliegen können. So können PQAV-Gruppen beziehungsweise PQAV - Beschichtungen aufweisende Festkörper als bevorzugt feinteilige Feststoffe Verwendung finden, die beispielsweise zu entkeimenden Flüssigphasen zugesetzt werden können, auf zu entkeimende Feststoffoberflächen verteilt und gewünschtenfalls dort verrieben werden können oder die auch im Zusammenhang mit einer unmittelbaren oder mittelbaren Wundversorgung verwendbar sind. Für die Behandlung von Gasphasen wiederum kann die Fixierung der unlöslichen PQAV in Filterbetten vorgesehen sein, wobei insbesondere auch Feuchtfilter Bedeutung haben können. Andere raumfüllende Ausgestaltungen der PQAV aufweisenden Festkörper sind beispielsweise entsprechend ausgerüstete Watte, Vließstoffe oder andere flächenartige Gebilde wie Tücher, die in vielgestaltigster Form auf den genannten Einsatz gebieten zur Behandlung von

geeignet, die in an sich bekannter Weise mit einem handelsüblichen Quaternierungsmittel, beispielsweise mit 3-Chlor-2-hydroxypropyltrimethylammoniumchlorid umgesetzt wird. Anstelle von Cellulose können unlösliche Cellulosederivate oder beliebige andere unlösliche und/oder unlöslich gemachte Natrustoffe und/oder Syntheseprodukte eingesetzt werden, die an ihrer Oberfläche reaktive Gruppen für eine solche Anbindung der polykationischen Elemente aufweisen. Geeignet sind beispielsweise der Cellulose vergleichbare andere polysaccharidartige Naturstoffe beziehungsweise entsprechend unlöslich gemachte Naturstoffderivate. Besonders geeignet können Guar-Abkömmlinge, insbesondere vernetzte Guar-Abkömmlinge sein. Aber auch ein teil- oder vollsynthetisches Material, das beispielsweise mit einer reaktiven vernetzten Polymerhülle eingeschlossen ist, entspricht den Voraussetzungen für die Herstellung von keimbindenden unlöslichen Hilfsmitteln nach der hier geschilderten Ausführungsform.

In einer anderen Ausführungsform wird von dem zuletzt angesprochenen Umhüllungsprinzip Gebrauch gemacht. Trägerstoffe beliebiger Ausgestaltung können mit vorgebildeten PQAV-Verbindungen in an sich bekannter Weise umhüllt werden, woraufhin diese flockungsaktive Materialschicht auf der Oberfläche der Feststoffpartikel in hinreichender Form zu immobilisieren ist. Hierdurch wird eine besonders große Variationsbreite in der Ausgestaltung der konkreten Erscheinungsform der keimbindenden Hilfsmittel ermöglicht. Einerseits ist die praktisch freie Wahl des unlöslichen Trägers mit seiner beliebigen Raumform gegeben, zum anderen besteht freie Wahlmöglichkeit in der Auswahl der polykationischen Ausrüstung und deren optimale Anpassung an das im Einzelfall gewünschte Arbeitsergebnis.

Die Trägerstoffe für die Ausrüstung mit PQAV-Komponenten können organischer und/oder anorganischer Natur sein, soweit sichergestellt ist, daß das Kombinationsmaterial unter Einsatzbedingungen hinreichend unlöslich ist. Die Verfestigung des vorgebildeten polykationischen Polymeren auf der Oberfläche solcher unlöslicher Träger kann unter Einsatz von chemischen und/oder physikalischen Bindungsprinzipien verwirklicht werden. So kann beispielsweise ein unlöslicher Träger gewählt werden, der salzbildende Gruppen, insbesondere Säuregruppen enthält, die dann bei der Beschichtung mit einem vorgebildeten PQAV-Polymeren zu einer Fixierung und Salzbildung führen.

Möglich ist aber auch der Auftrag entsprechender vorgebildeter polykationischer Polymerverbindungen auf die Feststoffoberfläche und deren anschließende Reaktion unter Molekülvergrößerung bis zur hinreichenden Unlöslichkeit. Diese chemische Reaktion ursprünglich löslicher oder hinreichend quellbarer polykationischer Polymerverbindungen kann beispielsweise mit üblichen Vernetzungsmitteln wie Epichlorhydrin oder Glyoxal vorgenommen werden, so daß letztlich auch auf diese Weise die gewünschten Hilfsmittel mit ihrer bindungsaktiven immobilisierten Ausrüstung auf der Oberfläche des unlöslichen Trägers entstehen.

Allgemeines chemisches Wissen ermöglicht damit, daß für den erfindungsgemäßen Zweck alle die aus dem Stand der Technik bekannten, insbesondere oligomeren und/oder polymeren polykationischen Komponenten verwendet werden können, die ursprünglich in löslicher Form vorliegen, dann aber in der geschilderten Weise in den unlöslichen Zustand überführt werden.

Aus der umfangreichen einschlägigen Literatur seien die folgenden Druckschriften beispielhaft benannt, deren Offenbarung hiermit ausdrücklich auch zum Gegenstand der Offenbarung der vorliegenden Erfindungsbeschreibung zur Struktur der PQAV gemacht wird: US-PSen 3 589 978, 3 632 559, 3 910 862, 4 157 388, 4 240 450 und 4 292 212, GB-PS 1 136 842, DE-AS 27 27 255, sowie die darin benannte US-PS 3 472 840.

Geeignete ursprünglich wasserlösliche oder auch wasserunlösliche PQAV im Sinne der Erfindung haben bevorzugt ein durchschnittliches Molgewicht von wenigstens etwa 200, vorzugsweise von wenigstens etwa 300 und insbesondere von wenigstens etwa 1000. Die obere Grenze der PQAV ist im Grunde bedeutungslos und liegt beispielsweise bei 10 Millionen oder auch bei weit höheren Werten. Verständlich ist das aus der erfindungsgemäß geforderten Bedingung der Unlöslichkeit der PQAV. Ist diese sichergestellt, sind dem Molekulargewicht nach oben keine Grenzen gesetzt.

Nach geeigneter, im folgenden geschilderter Aufbereitung für die Zwecke der Erfindung sind als zunächst wasserlösliche, dann aber auf einem unlöslichen Träger immobilisierte PQAV alle Polymeren geeignet, die entweder in der Polymerkette oder an die Polymerkette gebunden quartäre Ammoniumgruppen tragen. Solche quartären Ammoniumgruppen können sich auch von zyklisch gebundenem Stickstoff ableiten. Beispiele für solche quartäre Ammoniumgruppen sind entsprechende Glieder von 5- oder 6-gliedrigen Ringsystemen, z.B. von Morpholin-, Piperidin-, Piperazin-oder Indazol-Ringen. Zahlreiche Beispiele für solche wasserlöslichen PQAV sind z.B. in der US-PS 4 240 450 näher beschrieben.

Bevorzugt geeignet können Homo- oder Mischpolymerisate mit zyklischen Einheiten sein, wie sie im einzelnen aus der US-PS 3 912 808 bekannt sind. Handelsprodukte dieser Struktur sind z.B. Merquat®100 und Marquart®550 (Quaternium 41).

Weitere bevorzugt geeignete PQAV sind bespielsweise Celluloseether, deren Anhydroglucose-Einheiten

Flüssigphasen, harten Oberflächen, Gasphasen und/oder zur Behandlung verletzter und/oder unverletzter Bereiche des menschlichen oder tierischen Körpers Verwendung finden können. Wie bereits angegeben, kann insbesondere eine Kombination mit üblichen Desinfektionsmitteln vorgesehen sein, soweit hierdurch im Einzelfall die Fähigkeit der PQAV zur adsorptiven Mikroorganismen-Bindung nicht oder nicht substanziell beeinträchtigt wird.

Die Bindungsaktivität der erfindungsgemäß eingesetzten Hilfsmittel wird durch die Vielzahl von quartären Ammoniumgruppierungen an den unter Einsatzbedingungen unlöslichen Feststoffen sichergestellt, die wenigstens auf der Oberfläche dieser Feststoffkörper fixiert sind. Im einzelnen gelten für die Beschaffenheit solcher PQAV-modifizierter Festkörper die Angaben der genannten älteren Anmeldung P 36 44 579.7 (D 7816) zu den dort beschriebenen Flockungs- und Filtrierhilfsmitteln sinngemäß. Diese Offenbarung der genannten älteren Anmeldung wird hiermit ausdrücklich auch zum Gegenstand der Offenbarung der vorliegenden technischen Lehre gemacht. Ohne Anspruch auf Vollständigkeit gilt dementsprechend das Nachfolgende zur Beschaffenheit der PQAV-Komponenten, wobei sich die im nachfolgenden gegebene allgemeine technische Lehre jeweils sinngemäß in die vom Anwendungszweck her bestimmte spezielle Raumgestaltung aufgrund des allgemeinen Fachwissens umsetzen läßt.

Die im Sinne der Erfindung unlöslichen, gegenüber Keimen bindungsaktiven PQAV-Komponenten können in ihrer Gesamtheit das zur Keimbindung eingesetzte Material bilden. In diesem Fall ist das als unlöslicher Fänger eingesetzte Mittel als Ganzes aus entsprechender unlöslicher PQAV gebildet.

In einer weiteren und in der Regel wichtigeren Ausführungsform wird jedoch nur ein Teil des unlöslichen Materials mit Bindungsfähigkeit gegenüber Keimen durch die bindungsaktiven PQAV-Komponenten gebildet. Der Rest ist ein Trägermaterial, das sich seinerseits durch die Unlöslichkeit in den zu behandelnden Medien auszeichnet, im übrigen aber in vielgestaltigster Weise variiert und modifiziert werden kann. Die bindungsaktive polykationische Materialschicht liegt in dieser Ausführungsform wenigstens an der Außenfläche des jeweiligen unlöslichen Feststoffkörpers vor. Der Trägerkern - in der bevorzugten Ausführungsform die Hauptmasse des keimbindenden Hilfsmittels -unterliegt bezüglich seiner chemischen und/oder physikalischen Beschaffenheit keinen Einschränkungen, solange unerwünschte Interaktionen mit den zu behandelnden Phasen, insbesondere Flüssigkeiten und/oder Gasphasen ausgeschlossen und das Erfordernis der Unlöslichkeit sichergestellt sind.

Wie in der genannten älteren Anmeldung im einzelnen beschrieben, kann beispielsweise für die Behandlung von Flüssigphasen ein solcher Trägerkern in vielgestaltigster Weise variiert werden. Durch Wahl des spezifischen Gewichtes des Trägerkerns kann Einfluß auf das Verhalten der Masse in der Flüssigphase genommen werden. Spezifisch schwere Trägermaterialien führen zum Absetzen, vergleichsweise leichte Trägerstoffe können das Aufschwimmen der Aktivmasse begünstigen.

Die Immobilisierung der flockungsaktiven PQAV-Gruppen an beziehungsweise auf dem unlöslichen Feststoff kann auf chemischem und/oder physikalischem Weg erfolgen. Voraussetzung ist lediglich, daß unter den jeweiligen Anwendungsbedingungen die hinreichende Immobilisierung dieser bindungsaktiven Materialschicht am unlöslichen Träger gewährleistet ist. Zu Herstellung der erfindungsgemäß eingesetzten Hilfsmittel zur Keimbindung gelten die nachfolgenden allgemeinen Angaben:

In einer ersten Unterteilung der erfindungsgemäß brauchbaren Materialien lassen sich zwei Ausgestaltungen unterscheiden. In der ersten Ausgestaltung ist der unlösliche Feststoffkörper in seiner Gesamtheit aus einer entsprechend unlöslichen oder unlöslich gemachten PQAV-Verbindung gebildet. Hier liegen entsprechende bindungsaktive Gruppierungen, also nicht nur an der Oberfläche, sondern auch im Inneren des jeweiligen Feststoffteilchens vor.

In der zweiten und im allgemeinen bevorzugten Ausführungsform liegt die bereits angesprochene Ausgestaltung vor, daß auf einem unlöslichen Feststoffträger die PQAV-Ausrüstung in bevorzugt dünner Schicht aufgetragen und hier immobilisiert ist. Für die Keimbindung ist insbesondere die Interaktion zwischen Keimen und der Feststoffoberfläche des erfindungsgemäß eingesetzten Reinigungsmitels bedeutungsvoll, so daß also eine entsprechende Ausrüstung der Oberfläche der in Feststoffphase eingesetzten Hilfsmittel hinreichend ist. Schon aus wirtschaftlichen Überlegungen wird in der Regel dieser Ausführungsform besondere Bedeutung zukommen.

Für diese zuletzt geschilderte Ausführungsform der oberflächlichen Ausrüstung der erfindungsgemäß verwendeten keimbindenden Hilfsstoffe lassen sich wieder zwei Ausgestaltungen unterscheiden. In der ersten Ausführungsform wird die PQAV-Oberflächenausrüstung dadurch erreicht, daß durch geeignete Anbindung eine Vielzahl der gewünschten polykationischen Gruppen an der Feststoffoberfläche hinreichend fest verankert wird. Am nachfolgenden Beispiel wird diese Ausführungsform aufgezeigt: Ein aufgrund seiner chemischen Konstitution reaktionsfähiger unlöslicher Grundstoff wird mit einem üblichen Quaternierungsmittel unter Anbindung von einer Vielzahl von quartären Ammoniumgruppen auf der Oberfläche des unlöslichen Reaktanten umgesetzt. Als unlöslicher Reaktant ist beispielsweise körnige oder pulvrige Cellulose

über Äthersauerstoff gebundene Substituenten mit quartären Ammoniumgruppen tragen. Solche Polymeren sind z.B. aus der US-PS 3 472 840 bekannt. Ein Handelsprodukt mit dieser Struktur ist z.B. das Polymer-JR®400.

Weitere besonders geeignetete kationische Polymeren sind z.B. die aus der US-PS 3 910 862 bekannten und z.B. unter der Handelsbezeichnung Gafquat®734 und 755 erhältlichen quartären Polyvinylpyrolidon-Copolymerisate und die aus der US-PS 4 157 388 bekannten und z.B. unter der Handelsbezeichnung Mirapol ®A15 erhältlichen quartären polymeren Harnstoffderivate. Geeignete Copolymerisate mit polykationischem Charakter sind auch die in der offengelegten Europäischen Patentanmeldung 0 153 146 beschriebenen Polyacrylamid-Copolymeren, die insbesondere neben wenigstens 50 Mol% Acrylamid-Einheiten bis zu 50 Mol% eines quarternisierten Aminoalkylesters von Acrylsäure oder Methacrylsäure enthalten. Diese Copolymeren sind wasserlöslich.

Als Ausgangsmaterial bevorzugt geeignete PQAV sind solche Verbindungen, die in fester Form Schwierigkeiten bei der Auflösung in Wasser bereiten. Solche kationischen Polymeren sind vor allem die beispielsweise aus der GB-PS 1 136 842 bekannten kationischen Polygalactomannan-Derivate.

Galactomannane sind Polysaccharide, die in den Endospermzellen vieler Leguminosensamen vorkommen, die aber im industriellen Maßstab nur aus Johannesbrotkernmehl (locust bean gum), Guar-Gummi (guar gum) und Tara-Gummi (tara gum) gewonnen werden. Sie sind aufgebaut aus einer linearen Mannan-Hauptkette, bestehend aus beta-(1.4)-glycosidisch verknüpften Mannopyranosebausteinen, an die als Verzweigung einzelne Galactopyranose-Reste in alpha-(1.6)-glycosidischer Bindung fixiert sind. Die einzelnen Polygalactomannane unterscheiden sich hauptsächlich durch das Mannose-Glactose-Verhältnis. Die kationischen Derivate der Polygalactomannane werden hergestellt durch Umsetzung von Hydroxylgruppen des Polysaccharids mit reaktiven quartären Ammoniumverbindungen. Als reaktive quartäre Ammoniumverbindungen eignen sich z. B. solche der allgemeinen Formel

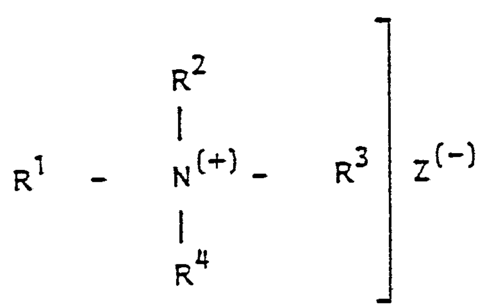

in der $R^1$, $R^2$ und $R^3$ z. B. Methyl- oder Ethylgruppen und $R^4$ eine Epoxyalkylgruppe der Formel

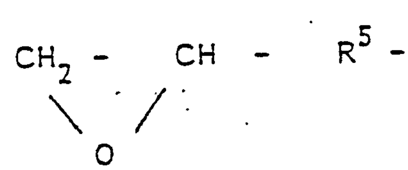

oder eine Halohydringruppe der Formel

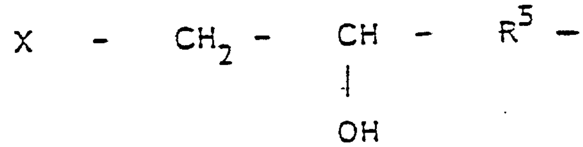

bedeuten und in welchen $R^5$ eine Alkylengruppe mit 1 - 3 C-Atomen, X = Chlor oder Brom und Z ein Anion wie z. B. Chlorid, Bromid, Jodid oder Hydrogensulfat ist. Der Substitutionsgrad sollte wenigstens 0,01 und bevorzugt wenigstens 0,05 sein und liegt typischerweise zwischen 0,05 und 0,5. Ein besonders geeignetes quartäres Ammoniumderivat eines Polygalactomannans ist z. B. das Guar-hydroxypropyl-trimethylammoniumchlorid, welches an die Sauerstoffatome der Hydroxylgruppen des Polysaccharids gebundene kationi-

sche Gruppen der Formel

$- CH_2 - CH(OH) - CH_2 - N^{(+)} - (CH_3)_3 ] Cl^{(-)}$

trägt. Solche kationischen Guar-Derivate sind z. B. unter der Handelsbezeichnung "Cosmedia Guar C 261" auf dem Markt. Der Substitutionsgrad (DS) von Cosmedia Guar C 261 liegt bei etwa 0,07. Auch die Handelsprodukte "Jaguar C-13" (DS = 0,11 - 0,13) und "Jaguar C 13 S" (DS = 0,13) gehören diesem Typ an.

Ein wichtiges Einsatzgebiet der hier aus dem Stand der Technik beispielhaft zitierten PQAV-Komponenten ist das Gebiet kosmetischer Präparate, insbesondere zur Behandlung bzw. Konditionierung von Haar. Er ist ein bekanntes Charakteristikum der PQAV, daß sie zum Aufziehen auf Feststoffoberflächen befähigt sind, wobei diese Fähigkeit insbesondere auch in Gegenwart üblicher tensidischer Komponenten gegeben sein kann. Je nach Konstitution ist dabei das Aufziehvermögen und die Haftfestigkeit der PQAV auf der Feststoffunterlage unterschiedlich stark ausgeprägt. Für das Verhalten der PQAV unter der Einwirkung wässrig-tensidischer Bäder kann allerdings auch die Interaktion mit insbesonderer aniontensidischen Komponenten ausschlaggebende Bedeutung haben. Bei stöchiometrischen oder annähernd stöchiometrischen Mengen der aniontensidischen Komponenten bildet sich in aller Regel an der quartären Ammoniumgruppe das entsprechende Aniontensidsalz aus. Solche PQAV-Aniontensidsalze zeigen im allgemeinen eine stark verringerte Wasserlöslichkeit. Es bilden sich entsprechende Niederschläge, vergleiche hierzu beispielsweise die deutsche Offenlegungsschrift 22 42 914.

Bekannt ist allerdings in diesem Zusammenhang weiterhin, daß durch Überschüsse, insbesondere beträchtliche Überschüsse des Aniontensides, eine Wiederauflösung der primär ausgefällten PQAV-Aniontensidsalze bedingt sein kann, vergleiche hierzu die Veröffentlichung in "Seifen/Öle/Fette/Wachse" 1985, 529-532 und 612-614. Beschrieben wird hier insbesondere die Bildung solubilisierender Komplexe mit Micellstruktur des Aniontensid/PQAV-Komplexes bei einem Überschuß der Aniontenside. Für die erfindungsgemäßen Zwecke kann also eine Immobilisierung solcher prinzipiell löslicher PQAV-Komponenten mittels Salzbildung über die quartäre Ammoniumgruppierung im Sinne entsprechender Aniontensid-Salze nur dann in Betracht kommen, wenn unter den zu erwartenden Anwendungsbedingungen nicht mit Überschüssen von zusätzlichen Aniontensiden in den zu reinigenden Flüssigphasen zu rechnen ist. In der Praxis wird von dieser Möglichkeit allerdings in einer ganzen Reihe von Fällen Gebrauch gemacht werden können.

Für eine weiterführende Verfestigung der vorgebildeten polykationischen Komponenten auf einem Trägerkern stehen die bereits erwähnten Prinzipien der chemischen Anbindung bzw. Molekülvergrößerung bis zum Zustand der Unlöslichkeit, insbesondere mittels difunktioneller Reaktionskomponenten und/oder die salzförmige Anbindung zur Verfügung.

In dieser zuletzt genannten Ausführungsform erfolgt die Verfestigung einer polymeren polykationischen Beschichtung auf einem formgestalteten Grundkörper dadurch, daß in bzw. an der Oberfläche des Grundkörpers anionische Gruppierungen ausgebildet werden bzw. vorliegen. Beispiele für solche anionischen Gruppierungen sind Carboxylgruppen, die beispielsweise durch Carboxymethylierung in die Oberfläche des Grundkörpers eingeführt werden können, oder andere Säuregruppierungen wie Sulfosäurereste. Weiterführende Angaben finden sich beispielsweise in der US-PS 3 694 364. Die kationische PQAV-Beschichtung bindet sich dann salzartig an diese Gegenionen der Matrix, so daß auf diese Weise der verfestigte Verbund zwischen unlöslichem Festkörper und aufgetragener PQAV-Schicht geschaffen wird. Lediglich beispielhaft für diese Ausführungsform sei ein Grundkörper auf Basis von Cellulosefasern gennant, bei dem freie Carboxylgruppen in das Celluosemolekül eingeführt worden sind. Möglich ist das beispielsweise auf zwei verschiedenen Wegen:

- durch physikalische Inkorporation von Carboxylgruppen tragenden Verbindungen in die Viskose, d.h. in eine als Cellu losexanthogenat gelöste Cellulose unter Bildung sogenannter inkorporierter Cellulosefasern oder
- durch chemische Umsetzung (Verätherung) der faserbildenden Cellulose der Carboxylgruppen tragenden Reagentien unter Bildung von einheitlich mit z.B. Carboxyalkylgruppen der Formel

$- (CH_2)_n - COOH,$

in der n einen Wert von 1 bis 3 haben kann, modifizierten Cellulosefasern.

Die physikalische Inkorporation Carboxylgruppen tragender Verbindungen in die Viskose wird z.B. durch Beimischen von Alkalisalzen von Acrylsäure-Homopolymerisaten, Acrylsäure-Methacrylsäure-Copolymerisaten, Methylvinylether-Maleinsäureanhydrid-Copolymerisaten, Alginsäure oder Carboxymethylcellulose zur Viskoselösung und anschließendes Verspinnen in üblicher Weise in ein Fällungsbad erreicht. Handelsprodukte auf Basis solcher Cellulosefasern als auch auf Basis der mit Carboxymethylgruppen modifizierten Fasern sind für viele Anwendungszwecke erhältlich. In der hier geschilderten Ausführungsform werden solche Fasern bzw. daraus hergestellte Trägermaterialien mit einer unlöslichen PQAV-Schicht überzogen und damit für den Einsatzzweck unlösbar verbunden.

Die Schichtstärke der bindungsaktiven Ausrüstung auf der Oberfläche des unlöslichen keimbindenden Hilfsmittels im Sinne der Erfindung kann ansich in beliebigen Grenzen gehalten werden. Berücksichtigt man, daß weitgehend Wechselwirkungen über Oberflächenkräfte für die Bindungskräfte entscheidend sein können, dann bietet sich schon aus Gründen der Wirtschaftlichkeit ein möglichst dünner Auftrag der aktiven Ausrüstung an.

Im Falle der unmittelbaren chemischen Substitution der Feststoffoberfläche, beispielsweise mit einer Vielzahl von quartären Ammoniumgruppierungen, ergibt sich von vornherein eine dünne Schichtstärke, die bis in den Bereich monomolekularer Schichten reichen kann. Der Substitutionsgrad in diesen Außenschichten kann bis zur theoretischen Grenzmöglichkeit reichen, wenngleich sich - umgerechnet auf das Gesamtmaterial - vergleichsweise niedere Substitutionsgrade errechnen, die beispielsweise 1 und vorzugsweise 0,5 im Falle der Cellulose oder vergleichbarer Polysaccharide bzw. Polysaccharidderivaten nicht überschreiten. Schon vergleichsweise niedere Substitutionsgrade im Bereich von etwa 0,01 bis 0,5 und insbesondere solche im Bereich von 0,1 bis 0,4 führen hier zu hochwirksamen Flockungshilfsmitteln im Sinne der Erfindung.

Werden vorgebildete Polymere auf die Oberfläche von unlöslichen Feststoffen aufgebracht und hier immobilisiert, so wird die Dicke der Schichtlage des Polymeren Werte von etwa 1mm und bevorzugt etwa 0,5 mm nicht überschreiten. Insbesondere können auch wesentlich dünnere Schichtlagen eingesetzt werden, die beispielsweise unter 0,1 mm liegen und beispielsweise im Schnitt im Bereich von 0,001 bis 0,05 mm Stärke ausgebildet sein können. Aber auch noch dünnere durchschnittliche Schichtdicken führen zu wirkungsvollen Ergebnissen. Die Anzahl der quartären Ammonium-Gruppen bestimmt sich durch die Zusammensetzung des vorgebildeten Polymeren. Werden beispielsweise Copolymerisate eines Monomeren mit einer quartären Ammoniumgruppierung im Molekül mit Comonomeren, die von diesen reaktiven Gruppen frei sind, eingesetzt, so können beispielsweise Mischungsverhältnisse dieser Monomertypen von 10 : 90 bis 90 : 10 und insbesondere 10 bis 60 Anteile des quartäre Ammoniumgruppen enthaltenden Monomers auf 90 bis 40 Anteile des stickstofffreien Comonomeren zum Einsatz kommen.

Als Trägerstoffe für die unmittelbare oder mittelbare Ausrüstung mit den polykationischen Komponenten kommen beliebige unlösliche organische und/oder anorganische Trägermaterialien in Betracht, die praktisch auch beliebige Formgestaltung aufweisen können. In einer bevorzugten Ausführungsform der Erfindung sind die Hilfsmittel der Erfindung pulvrig bis körnig, oder aber in Form von Faserstoffen ausgebildet. Pulvrig bis körniges Material hat in der Regel eine Teilchengröße nicht über 5 mm und insbesondere nicht etwa über 1 mm (durchschnittliche Teilchengröße). Feinteilige Materialien können besonders geeignet sein beispielsweise solche des Bereiches von etwa 0,5 bis 0,01 mm durchschnittliche Teilchengröße. Je feiner die Teilchengröße wird, umso größer wird die zur Verfügung gestellte Feststoffoberfläche - jeweils mengenmäßig gleiche Beträge des Mittels vorausgesetzt. Hieraus wird verständlich, daß insbesondere die feineren Materialien mit ihrer größeren Oberflächen bevorzugte Bedeutung haben können. Geeignete Oberflächen der Materialien liegen beispielsweise im Bereich von wenigstens etwa 0,1 $m^2/g$, insbesondere im Bereich von wenigstens etwa 0,5 $m^2/g$ und bevorzugt bei wenigstens etwa 1 $m^2/g$.

Als anorganische Trägermaterialien eignen sich zur Ausrüstung mit der polykationischen Ausrüstung alle anorganischen Komponenten hinreichender Unlöslichkeit in den zu behandelnden Medien. Ein wichtiges Einsatzgebiet solcher Hilfsmittel ist beispielsweise die Entkeimung von wässrigen Phasen, so daß also insbesondere hinreichend wasserunlösliche Mineralstoffe in dieser Klasse der anorganischen Trägermaterialien besondere Bedeutung haben können. Geeignet sind beispielsweise wasserunlösliche Silikatverbindungen wie Calcium-, Alumo- und/oder Magnesiumsilikate natürlichen und/oder synthetischen Ursprungs. In Betracht kommen hier insbesondere auch quellfähige Materialien von der Art natürlicher und/oder synthetischer montmorillonitartiger Verbindungen, ebenso wie nicht-quellende Alumosilikate von der Art synthetischer kristalliner Zeolithe, insbesondere der heute in großem Umfang eingesetzte Phosphataustauschstoff Zeolith A. Andere Beispiele für geeignete anorganische Materialien sind unter den Verfahrensbedingungen unlösliche Metalloxide und/oder Hydroxide bzw. entsprechende Metallsalze. Beispiele für Metalloxide bzw. Hydroxide sind etwa Kieselsäure, Aluminiumoxid, Magnesiumoxid und dergleichen. Als Beispiel für hinreichend wasserunlösliche Metallsalze sind Verbindungen wie Calciumcarbonat, Calciumsulfat und dergleichen zu nennen. Geeignete Trägermaterialien sind aber beispielsweise auch unlösliche mineralische Abfallstoffe aus technischen Verfahren, die bisher als unverwertbar angesehen und beispielsweise über Deponielagerung beseitigt werden. Ein besonderer Vorteil kann in der Verwendung von sauren bzw. polyanionisch ausgebildeten anorganischen Trägermaterialien liegen, wie sie beispielsweise im Fall der Alumosilikate, der Schichtsilikate oder des Kieselsäure-Gels gegeben sind. Auch hier ist durch die Möglichkeit der zusätzlichen ionischen Beeinflußung zwischen der polykationischen Beschichtungsmasse und dem anorganischen Grundkörper mit seiner gegensinnigen Ladung eine besonders feste Verankerung der keimbindenden Schicht gewährleistet.

In einer bevorzugten weiteren Ausführungsform werden allerdings solche Trägermaterialien für die Beschichtung mit der PQAV verwendet, die eine einfache Vernichtung der mit den Keimen besetzten verbrauchten Massen gewährleistet. Hier eignen sich insbesondere brennbare Trägermaterialien beispielsweise aus Cellulose oder celluloseartigen Produkten. Hiermit bietet sich eine besonders einfache Entsorgung für die Vernichtung der verkeimten Sammlermassen an.

Die quartäre Ammoniumgruppe in den erfindungsgemäß verwendeten Massen auf PQAV-Basis enthält bevorzugt 1 bis 3 niedere Alkylreste mit jeweils 1 bis 6, insbesondere 1 bis 3 und bevorzugt 1 bis 2 C-Atomen. Besondere Bedeutung kann der quartären Ammoniumgruppierung zukommen, die 1 bis 3 Alkylreste aufweist, die insbesondere Methyl- und/oder Ethylreste sind. Bevorzugt kommen solche PQAV zum Einsatz, die als bindungsaktive QAV-Gruppe Trimethylammoniumreste enthält.

Als Gegen-Ion können die üblichen salzbildenden Gruppen vorliegen, wie sie aus der Herstellung solcher PQAV-Komponenten anfallen. Als Beispiele sind hier Chlorid und/oder Sulfat zu nennen.

In einer weiteren wichtigen Ausführungsform der Erfindung werden allerdings die Mikroorganismen bindenden Hilfsmittel dadurch weitergehend modifiziert, daß vor ihrem Einsatz gezielt eine Konditionierung bzw. Aktivierung der Feststoffoberfläche vorgenommen wird. Hierzu wird von der Möglichkeit Gebrauch gemacht, die salzbildenden Gegenkomponenten zu den polykationischen Gruppierungen auszutauschen und damit gezielt neue funktionelle Elemente - über Salzbindung angebunden - in die bindungsaktive Schicht einzutragen.

In dieser Ausführungsform kann es besonders bevorzugt sein, den polykationischen Festkörper vor seinem Einsatz mit Netzmitteln zu behandeln. Geeignete Netzmittel sind tensidische Komponenten aus der Wasch- und Reinigungstechnik. Hierbei sind insbesondere anionische Tenside, nichtionische Tenside und/oder amphotere Tenside besonders geeignet.

Anionische Tenside bilden, wie bereits angegeben, mit quartären Ammoniumgruppierungen in stöchiometrischen Verhältnissen Salze. Gleichzeitig besteht aber nach der herrschenden Vorstellung die Möglichkeit, daß sich weitere tensidische Komponenten unter micellartiger Anlagerung an das salzartig gebundene Tensidmolekül anlagern. Bei den nicht hinreichend verfestigten polykationischen Komponenten - beispielsweise bei einer vorgebildeten polymerern PQAV - könnte das zur Wiederauflösung der bindungsaktiven Ausrüstung führen. In den erfindungsgemäß eingesetzten Hilfsmitteln ist das nicht der Fall, weil das physikalische Zustandsbild durch die Hauptmasse des Formkörpers geprägt wird, der insbesondere durch seine Unlöslichkeit charakterisiert ist. Hier wird ersichtlich, daß sich über eine solche Reaktion der polykationischen Ausrüstung mit tensidischen Komponenten die Möglichkeit erschließt, variierte bindungsaktive Schichten zu schaffen.

Die zur Modifizierung eingesetzten Netzmittel können - bezogen auf die jeweils vorliegenden basischen, stickstoffenthaltenden Gruppen in unterstöchiometrischen Mengen oder auch in überstöchiometrischen Mengen eingesetzt werden. Bevorzugt werden wenigstens etwa 0,5 Äquivalente an tensidischer Komponente und insbesondere wenigstens etwa 0,9 Äquivalente an tensidischer Komponente - bezogen auf basischen Stickstoff - verwendet. Der Einsatz etwa stöchiometrischer Mengen kann zweckmäßig sein. Überstöchiometrische Mengen können bis zu einem Mehrfachen, beispielsweise bis zum 10-50fachen, vorzugsweise bis etwa zum 5fachen der stöchiometrischen Menge eingesetzt werden.

Eine besonders einfache Konditionierung des erfindungsgemäß eingesetzten Hilfsmittels erhält man dadurch, daß man das beispielsweise körnige oder pulverförmige polykationisch ausgerüstete unlösliche Material in einer wässrigen Tensidlösung aufschlemmt, die die jeweils gewünschten Tensidkomponenten im Überschuß enthält und dann nach hinreichender Reaktionszeit -beispielsweise 1 bis 60 Minuten - die Feststoffphase von der wässrigen Phase abtrennt und gewünschtenfalls anschließend auswäscht. Die so gereinigten Komponenten können unmittelbar eingesetzt werden, sie können aber auch getrocknet werden, um dann zu einem späteren Zeitpunkt zum Einsatz zu kommen.

Diese weiterführende Behandlung ist jedoch keine zwingende Voraussetzung für die Wirksamkeit der erfindungsgemäß verwandten Hilfsmittel. Es werden im Gegenteil, beispielsweise mit tensidisch nicht vorbehandeltem PQAV-Komponenten der hier geschilderten Art, hervorragende Wirkungen erzielt.

Im wässrigen System liegen bevorzugt pH-Werte im Bereich von etwa 5 über neutral bis stark alkalisch vor, wobei der pH-Bereich von etwa 5,5 bis 13,5 besonders geeignet sein kann. Für die Anwendung der erfindungsgemäßen Hilfsmitel erschließt sich damit der breite Bereich schwach saurer, neutraler, schwach alkalischer bis stark alkalischer wässriger Phasen. In diesen Flüssigphasen vorliegende oder darin eingetragene Mikroorganismen, insbesondere Bakterien und/oder Hefen werden an den erfindungsgemäß eingesetzten Hilfsmitteln wirkungsvoll adsorbiert. Die adsorptive Bindekraft besonders geeigneter unlöslicher PQAV-Komponenten gegenüber den Mikroorganismen kann dabei so stark ausgeprägt sein, daß eine mehrschichtige Belegung der PQAV-Oberfläche mit Mikroorganismen festgestellt werden kann. Aber auch schon die einschichtige oder auch nur partielle Belegung der PQAV-Oberfläche mit den zu bindenden Keimen

kann zur wirkungsvollen Reduzierung der Keime in den zu behandelnden Medien führen.

Zur Bindung der Keime an die PQAV-Oberfläche bedarf es lediglich des hinreichenden Kontaktes zwischen Keim und PQAV-Feststoff. Die Fixierung des Einzelteilchens ist - wie mikroskopische Untersuchungen gezeigt haben - praktisch als Spontanreaktion anzusehen, so daß beträchtliche Reduzierungen der Keimgehalte beispielsweise in Flüssigphasen durch Behandlung solcher Phasen mit beschränkten Mengen an PQAV-Komponenten in vergleichsweise kurzen Zeiträumen von beispielsweise 0,5 bis 30 Minuten, insbesondere etwa 1 bis 10 Minuten erzielt werden können. In den nachfolgenden Beispielen findet sich hierfür charakteristisches Zahlenmaterial.

## Beispiele

### Beispiel 1

91,6 g (0,5 mol) Guarmehl mit einem Feuchtigkeitsgehalt von 11,6 Gew.-% wurden zusammen mit 32,75 g Natriumhydroxid-Pylver in 324 g 87 gew.-%igem wässrigen tert.-Butanol suspendiert. Die Suspension wurde unter intensivem Rühren in einer Stickstoffatmosphäre mit 162 g einer ca. 58 gew.-%igen wässrigen Lösung von 3-Chlor-hydroxypropyltrimethylammoniumchlorid (Quab 188, DEGUSSA) innerhalb von 10 Minuten versetzt. 5 Minuten wurde nachgerührt. Dann wurden 4,05 g Epichlorhydrin zugegeben und die Suspension anschließend auf eine Temperatur von 70 °C, bei der sie 4 Stunden gerührt wurde, erwärmt.

Nach Abkühlen auf Raumtempertur wurde das Reaktionsgemisch mit konzentrierter Salzsäure gegen Phenophtalein neutralisiert. Das wasserunlösliche Guarderivat wurde abfiltriert, gründlich mit Wasser gewaschen, mit Aceton entwässert und bei 60 °C im Vakuum getrocknet.

Erhalten wurden 107.5 g eines gelblichen Pulvers, dessen Stickstoffgehalt von ca. 2,42 Gew.-% einem Substitutionsgrad (MS) von ca. 0,38 entspricht.

Die eingesetzten Reagenzmengen entsprechen einem mol-Verhältnis von Guarmehl : Natriumhydroxid : Quab 188 : Epichlorhydrin von ca. 1 : 1,64 : 1 : 0,088.

In Parallelansätzen wird einmal destilliertes Wasser als solches mit Bakterien (Staph. aureus beziehungsweise Escherichia coli) beziehungsweise mit Hefe (Candida albicans) verkeimt, zum anderen werden entsprechende Ansätze in destilliertem Wasser vorgesehen, dem 0,5 Gewichtsprozent der zuvor beschriebenen unlöslichen PQAV-Komponente zugesetzt worden ist. Die dabei eingesetzten Ausgangs-Keimzahlen liegen im Fall der Bakterien im Bereich von 1 bis 3 x $10^8$/ml beziehungsweise bei 2 bis 3 x $10^7$/ml bei der Hefe. Anschließend werden die Versuchspräpärate geschüttelt.

Um den Einfluß der Schüttelintensität zu prüfen, werden in Parallelansätzen geeignete Volumina unterschiedlich stark geschüttelt, und zwar wie folgt:

1. 0,5 Minuten leichtes Rühren
2. 1 Minute Reagenzglasschüttler
3. 1 Minute Labor-Vibro-Mischer (der Chemap AG)
4. 10 Minuten Schüttelmaschine 250 rpm

Danach wurde der jeweilige Ansatz zur Sedimentation in ein Reagenzglas-artiges Gefäß überführt und nach 30 Minuten der Keimgehalt im klaren Überstand ermittelt. Die Ansätze ohne Zusatz von PQAV-Verbindung wurden jeweils als Negativkontrolle mitgeführt.

In der nachfolgenden Tabelle 1 ist jeweils die Restkeimzahl pro ml im Überstand der einzelnen Ansätze nach Sedimentation der PQAV-Komponente angegeben.

Tabelle 1

| Präparat | Staph. aureus Mischmethode | | | Escherichia coli Mischmethode | | | | Candida albicans Mischmethode | | |
|---|---|---|---|---|---|---|---|---|---|---|
| | 1. | 2. | 3. | 1. | 2. | 3. | 4. | 1. | 2. | 3. |
| mit PQAV-Zusatz | $9,8x10^5$ | $6,4x10^5$ | $6,4x10^5$ | $1,3x10^6$ | $1,0x10^6$ | $3x10^6$ | $3x10^3$ | $6x10^3$ | $9x10^1$ | $5x10^1$ |
| ohne PQAV-Zusatz | $3,2x10^8$ | $2,8x10^8$ | $2,6x10^8$ | $1,1x10^8$ | $1,0x10^8$ | $9x10^7$ | $1x10^8$ | $2,6x10^7$ | $2,6x10^7$ | $2,6x10^7$ |

Beispiel 2

Die Verfahrensmethodik des Beispiels 1 wird mit Hefekeime (Candida albicans) enthaltenden Wasserproben wiederholt, es werden jedoch abgewandelte PQAV-Komponenten zur Behandlung der verkeimten Wasserproben eingesetzt. Dabei gilt im Einzelnen:

PQAV-B ist ein der Komponente aus Beispiel 1 vergleichbares Guar-Präparat mit einem mittleren Substitutionsgrad (MS) von 0,165

PQAV-C ist ein entsprechendes Präparat auf Stärkebasis mit einem mittleren Substitutionsgrad (MS) von 0,06

PQAV-D ist wieder ein entsprechendes Stärkepräparat mit einem mittleren Substitutionsgrad (MS) von 0,15

Es werden die Mischmethoden zu 1. bis 3. aus Beispiel 1 in vergleichenden Untersuchungen eingesetzt. Auch in diesem Fall werden die PQAV-Komponenten jeweils in Mengen von 0,5 Gewichtsprozent verwendet.

Die Restkeimzahlen in den behandelten und durch Sedimentation gereinigten Flüssigphasen sind in der nachfolgenden Tabelle 2 zusammengefaßt, wobei zum Vergleich auch hier als Negativkontrolle jeweils ein Ansatz ohne PQAV-Substanz mitgeführt worden war.

Tabelle 2

| Präparat | Candida albicans Mischmethode | | |
|---|---|---|---|
| | 1. | 2. | 3. |
| PQAV-B | $4 \times 10^6$ | $7 \times 10^5$ | $5 \times 10^5$ |
| PQAV-C | $9 \times 10^6$ | $1,8 \times 10^6$ | $1,2 \times 10^4$ |
| PQAV-D | $3 \times 10^6$ | $3 \times 10^3$ | $2,0 \times 10^3$ |
| ohne PQAV | $2,6 \times 10^7$ | $2,6 \times 10^7$ | $2,6 \times 10^7$ |

Beispiel 3

In einer weiteren Versuchsreihe wurde die Abhängigkeit des Effektes der adsorptiven Keimbindung vom pH-Wert im Reaktionsansatz untersucht.

Dazu wurde die PQAV-Komponente des Beispiels 1 jeweils 0,5 %ig in verschiedenen Puffersystemen angesetzt:

pH 3 = Citronensäure/NaOH/NaCl
pH 5 = Na-acetat/Essigsäure
pH 7 = $KH_2PO_4/Na_2HPO_4$
pH 9 = Borax/Salzsäure
pH 11 = Borsäure/KCl/NaOH

Nach Beimpfen mit dem Bakterium E. coli wurden die Ansätze 1 Minute auf dem Reagenzglasschüttler

geschüttelt und nach weiteren 30 Minuten Sedimentation der Keimgehalt/ml im Überstand gemessen. Als Negativkontrollen wurden die einzelnen Pufferlösungen ohne Zusatz von PQAV-Komponente beimpft und nach entsprechender zeitlicher Verzögerung auf ihren Keimgehalt untersucht.

In der nachfolgenden Tabelle 3 sind die entsprechenden Keimzahlen wiedergegeben.

Tabelle 3

| pH-Wert im Ansatz | Keimzahl/ml in Kontrolle ohne Fänger | Versuchs-Ansatz mit Fänger | Keimreduktion um .. Zehnerpotenzen |
|---|---|---|---|
| 3,0 | $3 \times 10^7$ | $1 \times 10^7$ | 0,5 |
| 5,0 | $4 \times 10^7$ | $1 \times 10^7$ | 0,6 |
| 7,0 | $2 \times 10^7$ | $1 \times 10^4$ | 3,3 |
| 9,0 | $2 \times 10^7$ | $4 \times 10^4$ | 2,7 |
| 11,0 | $3 \times 10^7$ | $5 \times 10^5$ | 1,8 |

**Ansprüche**

1. Verwendung von unter Einsatzbedingungen unlöslichen und/oder auf einem unlöslichen Träger immobilisierten polyfunktionellen, quartären Ammoniumverbindungen (PQAV) zur adsorptiven Bindung von Mikroorganismen, insbesondere Hefen und/oder Bakterien.

2. Ausführungsform nach Anspruch 1 dadurch gekennzeichnet, daß die unlöslichen und/oder immobilisierten PQAV zur Entkeimung von Flüssigphasen, insbesondere von wäßrigen Flüssigkeiten, Gasphasen, insbesondere Raumluft, harten Oberflächen beziehungsweise zur Entkeimung verletzter oder unverletzter Bereiche des menschlichen oder tierischen Körpers, insbesondere zur Wundversorgung Verwendung finden.

3. Ausführungsform nach Ansprüchen 1 und 2 dadurch gekennzeichnet, daß die PQAV aufweisenden Festkörper als feinteilige Feststoffe, in Filterbetten immobilisiert und/oder auf anderen raumfüllenden Festkörpern wie Watte oder flächigen Materialien z.B. Tüchern, zum Einsatz kommen.

4. Ausführungsform nach Ansprüchen 1 bis 3 dadurch gekennzeichnet, daß die PQAV im etwa neutralen bis alkalischen Bereich, bevorzugt im pH-Bereich von etwa 6 bis 13 verwendet werden.

5. Ausführungsform nach Ansprüchen 1 bis 4 dadurch gekennzeichnet, daß PQAV aufweisende Feststoffe zum Einsatz kommen, die an ihrer Oberfläche chemisch und/oder physikalisch gebunden eine Vielzahl quartärer Ammoniumgruppen aufweisen, die insbesondere 1 bis 3 niedere Alkylreste, bevorzugt Methyl- und/oder Ethylreste, aufweisen.

6. Ausführungsform nach Ansprüchen 1 bis 5 dadurch gekennzeichnet, daß PQAV zum Einsatz kommen, die als bindungsaktive quartäre Ammoniumgruppe Trimethylammoniumreste enthält.

7. Ausführungsform nach Ansprüchen 1 bis 6 dadurch gekennzeichnet, daß wenigstens an ihrer Oberfläche quaternisierte oder mit vorgebildeter PQAV überzogene Feststoffe natürlichen und/oder synthetischen Ursprungs eingesetzt werden, die insbesondere verbrennbar sind.